# EUROPÄISCHE PATENTSCHRIFT

(11) **EP 0 957 833 B1**
(45) Veröffentlichungstag und Bekanntmachung des Hinweises auf die Patenterteilung: **23.10.2002**
(21) Anmeldenummer: 96929301.8
(22) Anmeldetag: 21.08.1996
(51) Int. Cl.: A61F 2/34

(54) **KUGELGELENK-ENDOPROTHESE**
ENDOPROSTHESIS WITH A BALL-AND-SOCKET JOINT
ENDOPROTHESE A JOINT A ROTULE

(30) Priorität: 25.08.1995 DE 29513694 U
(43) Veröffentlichungstag der Anmeldung: 24.11.1999
(73) Patentinhaber: Waldemar Link GmbH & Co., 22339 Hamburg (DE)
(72) Erfinder: KELLER, Arnold, D-23863 Kayhude (DE); LUBINUS, Philipp, D-24106 Kiel (DE)
(74) Vertreter: Glawe, Delfs, Moll & Partner
(86) Internationale Anmeldenummer: EP9603685
(87) Internationale Veröffentlichungsnummer: WO97007754

(56) Entgegenhaltungen:
- EP-A- 0 285 756
- DE-A- 2 024 583
- DE-A- 4 337 936
- US-A- 5 019 105

## Beschreibung

Es ist bekannt (EP-A 53794), die Gelenkpfanne einer Kugelgelenk-Endoprothese, beispielsweise einer Hüftprothese, mit einer Gleitfläche aus keramischem Werkstoff auszurüsten. Wenn der darin geführte Gelenkkopf im Falle einer sogenannten Subluxation sich teilweise aus der vorgesehenen Lagerposition entfernt, kann es zu Linienkontakt mit der Gleitflächenkante kommen, was zu Schäden sowohl am Gelenkkopf als auch an der Kante der Gelenkpfanne führen kann. Es wurde deshalb vorgeschlagen, der Kante der Gelenkgleitfläche einen Schutzring aus plastisch verformbarem Polyethylen vorzuschalten (EP-A 53794).

Dieser Schutzring ist normalerweise keinem Verschleiß unterworfen. Jedoch kann es geschehen, daß der den Gelenkkopf tragende Hals bei stark ausladender Bewegung an dem Polyethylenring anschlägt. Dies kann zu schädlichem Abrieb führen. Auch hat sich gezeigt, daß der Schutzring dabei verformt werden kann, so daß seine Schutzfunktion für den Keramikteil beeinträchtigt wird.

Diese Nachteile werden erfindungsgemäß dadurch vermieden, daß die Stirnseite des Schutzrings durch einen Abdeckring aus widerstandsfähigem Material abgedeckt ist. Bei diesem Material kann es sich um Metall handeln oder auch um einen widerstandsfähigen Kunststoff. Damit im Falle einer Subluxation die Oberfläche des Gelenkkopfs nicht an dem Abdeckring anschlagen und beschädigt werden kann, ist der Schutzring zweckmäßigerweise so ausgebildet, daß er den Innenrand des Abdeckrings überdeckt.

Die Erfindung wird im folgenden näher unter Bezugnahme auf die Zeichnung erläutert, die ein vorteilhaftes Ausführungsbeispiel veranschaulicht. Darin zeigen:
- Fig. 1: einen vergrößerten Längsschnitt durch eine Hüftgelenk-Endoprothese und
- Fig. 2: einen noch weiter vergrößerten Teilschnitt durch den Rand der Gelenkpfanne.

Die Hüftpfanne 1 umfaßt eine Fassung 2, einen Polyethylenkörper 3 und einen Keramikeinsatz 4. Die Fassung 2 ist äußerlich so gestaltet, daß sie sich leicht und dauerhaft im Knochen verankern läßt. Zu diesem Zweck ist sie beispielsweise mit Vorsprüngen 5 versehen. Wegen der Einzelheiten kann auf den Stand der Technik verwiesen werden.

Sie weist eine kegelstumpfförmige Innenform 6, 7 auf, der die Außenform des Polyethylenkörpers 3 entspricht. Diese kann nach Implantation der Fassung 2 im wesentlichen spielfrei darin eingesetzt und verankert werden. Auch dies ist bekannt.

Der Polyethylenkörper 3 hat eine kegelstumpfförmige Innenform 8, 9, der spielfrei, ggf. mit etwas Übermaß die Außenform des Keramikeinsatzes 4 entspricht. Dieser besteht aus einem physiologisch verträglichen, hochverschleißfesten Keramikmaterial, beispielsweise hochreiner Aluminiumoxydkeramik. Er bildet eine sphärische Gleitfläche 10, die der sphärischen Gestalt des darin gelagerten Gelenkkopfs 11 entspricht. Die sphärische Gleitfläche 10 erstreckt sich in jeder Richtung über etwa 180°; der Äquator der die Gleitfläche 10 bildenden sphärischen Fläche liegt irgendwo zwischen dem Beginn der Kantenrundung 12 und der Stirnfläche 13.

Auf die Stirnfläche 13 des keramischen Einsatzes ist ein Schutzring 14 aus hochdichtem Polyethylen oder ähnlichem nachgiebigem, vorzugsweise hinreichend elastischem Material aufgelegt, der wiederum größtenteils von einem Schutzring 15 aus geeignetem Metall, beispielsweise Titan, abgedeckt ist. Dieser ist im dargestellten Beispiel als Gewindering ausgebildet, der über ein Gewinde 16 mit dem Polyethylenkörper 3 zusammenwirkt, um den Keramikeinsatz 4 und den Schutzring 14 an Ort und Stelle zu sichern. Eine nicht dargestellte Sicherung kann vorgesehen sein, die das ungewollte Herausdrehen des Abdeckrings 15 verhindert.

Der Polyethylenkörper 3 bewirkt eine gleichmäßig verteilte Kraftübertragung von dem Keramikkörper 4 auf die Fassung 2 und verhütet dadurch schädliche Spannungskonzentrationen am Keramikkörper 4. Desgleichen verteilt der Schutzring 14 die von dem Abdeckring 15 herrührende Haltekraft unter Vermeidung von Spannungsspitzen auf die Stirnfläche 13 des Keramikkörpers 4.

Der - von der Gleitfläche 10 des Keramikkörpers 4 her gesehen - vorzugsweise jenseits des Äquators gelegene Schutzring 14 ragt innen bis etwa an diejenige gedachte sphärische Fläche vor, die die Gleitfläche 10 öffnungsseitig fortsetzt. Einerseits hat er zweckmäßigerweise ein wenig Spiel gegenüber dem Gelenkkopf 11, wenn dieser ordnungsgemäß an der Gleitfläche 10 anliegt, damit Polyethylenabrieb vermieden wird und die Gelenkflüssigkeit freien Zugang zum Gleitspalt hat. Andererseits ist es zweckmäßig, wenn sein Innendurchmesser ein wenig kleiner ist als der Maximaldurchmesser des Gelenkkopfs 11, um eine Kraftschwelle gegen das Herausgleiten des Gelenkkopfs aus der Gelenkpfanne zu bilden. Die meisten Subluxationen können auf diese Weise verhindert werden. Die Kraftschwelle soll aber geringer sein als die Kraft, bei der sich der Gelenkkopf 11 von dem ihn tragenden Konuszapfen 17 lösen kann.

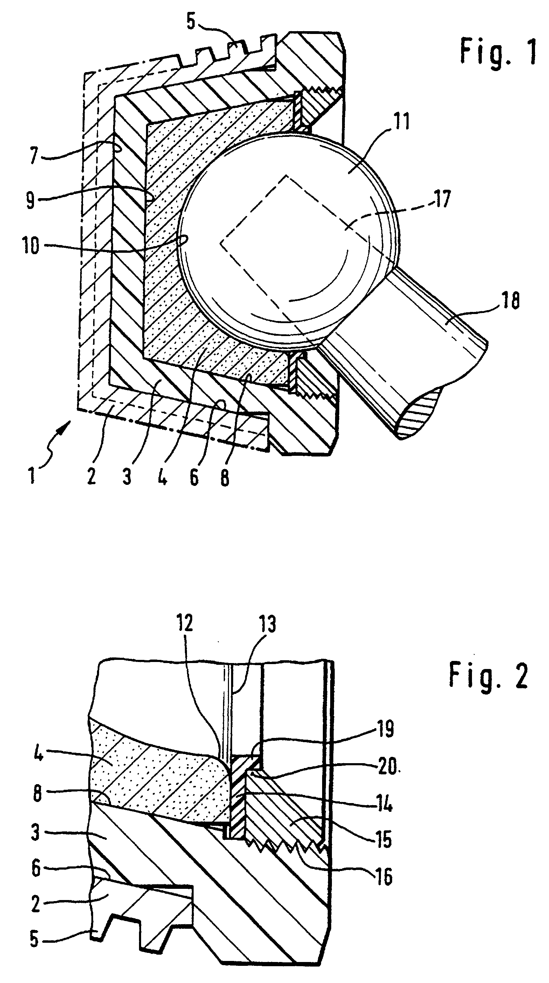

Der Abdeckring 15 schützt den Schutzring 14 vor möglicherweise schädigenden Stößen seitens des Kopfhalses 18. Aufgrund dieses Schutzes kann er mit stark reduzierten Maßen hergestellt werden. Seine Dicke liegt beispielsweise in der Größenordnung von einem Millimeter. An seinem inneren Rand weist er einen sich achsparallel nach außen erstreckenden Kragen 19 auf, der vor dem Innenrand 20 des Abdeckrings 15 liegt und dadurch verhütet, daß der Gelenkkopf 11 unmittelbar mit dem aus härterem Material bestehenden Abdeckring 15 in möglicherweise schädigenden Kontakt treten kann. Der Kragen 19 kann, wie man in Fig. 2 sieht, öffnungsseitig sogar etwas über den Rand des Abdeckrings 15 hinausragen. Zwar nimmt man dadurch in Kauf, daß der Kopfhals 18 an dem Kragen 19 anschlagen kann. Solange dieser elastisch ausweichen kann, ist dies jedoch unschädlich. Das elastische Ausweichen ist ihm dadurch möglich, daß zwischen der öffnungsseitigen Kantenrundung 12 des Keramikeinsatzes und dem Schutzring 14 ein Keilspalt sich öffnet, in den hinein der innere Rand des Schutzrings in einem solchen Fall elastisch zurückweichen kann.

## Patentansprüche

1. Endoprothese, die einen Gelenkkopf (11) und eine Gelenkpfanne (1) umfaßt, deren Gelenkgleitfläche (10) von einem Teil (4) aus keramischem Werkstoff gebildet ist und deren Öffnungskante (12) von einem Schutzring (14) aus nachgiebigem Material wie Polyethylen abgedeckt ist, **dadurch gekennzeichnet, daß** die Stirnseite des Schutzrings (14) durch einen Abdeckring (15) aus widerstandsfähigem Material abgedeckt ist.

2. Endoprothese nach Anspruch 1, **dadurch gekennzeichnet, daß** der Innenrand (20) des Abdeckrings (15) von dem Schutzring (14, 19) überdeckt ist.

3. Endoprothese nach Anspruch 1 oder 2, **dadurch gekennzeichnet, daß** die Öffnungskante (12) des keramischen Teils (4) abgerundet ist.

4. Endoprothese nach Anspruch 3, **dadurch gekennzeichnet, daß** die Abrundung (12) einen Keilspalt zwischen dem keramischen Einsatz (4) und dem Schutzring (14) bildet.

## Claims

1. Endoprosthesis which comprises a ball (11) and a socket (1) whose slide surface (10) is formed by a part (4) made of ceramic material, and whose opening edge (12) is covered by a protective ring (14) made of resilient material, such as polyethylene, **characterized in that** the front side of the protective ring (14) is covered by a covering ring (15) made of resistant material.

2. Endoprosthesis according to Claim 1, **characterized in that** the inner edge (20) of the covering ring (15) is covered by the protective ring (14, 19).

3. Endoprosthesis according to Claim 1 or 2, **characterized in that** the opening edge (12) of the ceramic part (4) is rounded.

4. Endoprosthesis according to Claim 3, **characterized in that** the rounding (12) forms a wedge-shaped gap between the deramic insert (4) and the protective ring (14).

## Revendications

1. Endoprothèse qui comprend une tête d'articulation (11) et une cavité articulaire (1) dont la surface de glissement (10) de l'articulation est formée par un élément (4) en matière céramique et dont le bord d'ouverture (12) est recouvert par un anneau de protection (14) en matière souple tel que le polyéthylène, **caractérisée en ce que** le côté frontal de l'anneau de protection (14) est recouvert par un anneau de couverture (15) en matière résistante.

2. Endoprothèse selon la revendication 1, **caractérisée en ce que** le bord intérieur (20) de l'anneau de couverture (15) est recouvert par l'anneau de protection (14, 19).

3. Endoprothèse selon la revendication 1 ou 2, **caractérisée en ce que** le bord d'ouverture (12) de l'élément céramique (4) est arrondi.

4. Endoprothèse selon la revendication 3, **caractérisée en ce que** la partie arrondie (12) forme une fente en coin entre l'insert céramique (4) et l'anneau de protection (14).
